# EUROPEAN PATENT SPECIFICATION

(11) **EP 2 475 414 B1**
(45) Date of publication and mention of the grant of the patent: **30.05.2018**
(21) Application number: 10759997.9
(22) Date of filing: 17.08.2010
(51) Int. Cl.: A61M 16/16

(54) **HUMIDIFIER WITH WIRELESS TEMPERATURE SENSING**
BEFEUCHTER MIT DRAHTLOSER TEMPERATURERFASSUNG
HUMIDIFICATEUR AVEC DÉTECTION DE TEMPÉRATURE SANS FIL

(30) Priority: 11.09.2009 US 241513 P
(43) Date of publication of application: 18.07.2012
(73) Proprietor: Koninklijke Philips N.V., 5656 AE Eindhoven (NL)
(72) Inventor: JACOB, Lawrence, Anthony, Briarcliff Manor, NY 10510-8001 (US)
(74) Representative: van Velzen, Maaike Mathilde
(86) International application number: PCT/IB2010/053714
(87) International publication number: WO 2011/030251

(56) References cited:
- WO-A1-2006/126900
- WO-A1-2008/091164
- US-A- 4 098 853
- US-A- 5 031 612
- US-A1- 2003 066 526
- US-A1- 2004 234 254

## Description

The present invention relates to temperature sensing devices of a humidifier reservoir.

Various breathing systems use humidifiers to humidify the air being provided to a user. For example, positive airway pressure (PAP) machines provide a continuous positive airway pressure to a user via a suitable user interface (e.g., a mask) for various medical reasons (e.g., to treat sleep apnea). Humidifiers are frequently provided between or integral with the PAP machine and the user interface in order to humidify the otherwise relatively-dry compressed air generated by the PAP machine.

WO 2008/091164 A1 discloses a humidification apparatus having RFID tag sensor at patient end of gas pathway. WO 2006/12690 A1 discloses a breathing assistance apparatus. US 2003/0066526 A1 discloses an apparatus for delivering humidified gases.

Typically, a temperature sensor is operatively connected to a humidifier reservoir to sense the temperature of the liquid (e.g., distilled water) in the humidifier reservoir, and a signal indicative of the temperature is transmitted via a mechanical or electrical connection between the humidifier reservoir and the base unit. Because the humidifier reservoir is constantly being detached from the base unit to be cleaned and refilled with liquid, there may be inaccuracies in the temperature received by the base unit due to the accumulated dirt or wear and tear of the mechanical or electrical connection between the humidifier reservoir and the base unit. The present invention addresses such deficiencies of the existing systems.

One aspect of the invention provides a respiration humidifier as defined in claim 1, the humidifier having a base unit and a detachable chamber constructed and arranged to hold a reservoir of liquid. The humidifier further includes a sensor carried by the chamber and operatively configured to sense a condition within the chamber, the condition being a temperature of the liquid or the humidity of the gas in the chamber. The humidifier also includes a radiation signal transmitter carried by the chamber and operatively associated with the sensor. The sensor is a sensor separated from the radiation signal transmitter. The sensor is operatively connected at a distance to the radiation signal transmitter via a connector. The transmitter is arranged to send wirelessly a radiation signal indicative of the sensed condition. The humidifier further includes a radiation signal receiver carried by the base unit and arranged to receive the radiation signal from the transmitter. The humidifier further includes a control unit that controls the condition based at least in part on the radiation signal received by the radiation signal receiver. The transmitter is arranged to be contactlessly powered by the receiver when the chamber is attached to the base unit and to be powered off when the chamber is detached from the base unit.

Another aspect of the invention provides a method for measuring a condition of a respiration humidifier comprising a base unit and a detachable chamber constructed and arranged to hold liquid as defined in claim 7. The method includes the step of sensing the condition within the chamber via a sensor carried by the chamber, the condition being a temperature of the liquid or the humidity of the gas in the chamber. The method also includes the steps of wirelessly sending a radiation signal indicative of the sensed condition with a radiation signal transmitter carried by the chamber and receiving the radiation signal indicative of the sensed condition with a radiation signal receiver. The sensor is a sensor separated from the radiation signal transmitter. The sensor is operatively connected at a distance to the radiation signal transmitter via a connector. The method further includes the step of controlling the condition based at least in part on the radiation signal received by the radiation signal receiver. The transmitter is arranged to be contactlessly powered by the receiver when the chamber is attached to the base unit and to be powered off when the chamber is detached from the base unit.

Another aspect of the invention provides a system for delivering a flow of gas to an airway of a user. The system includes a gas flow generator that generates a flow of gas and a user circuit coupled to the gas flow generator and adapted to communicate the flow of gas to an airway of a user. The system also includes a humidifier operatively connected to the user circuit to elevate the humidity of the gas in the user circuit to a circuit humidity level. The humidifier includes a base unit and a detachable chamber constructed and arranged to hold a reservoir of liquid. The system further includes a sensor operatively configured to sense a condition within the chamber, the condition being a temperature of the liquid or the humidity of the gas in the chamber. The system also includes a radiation signal transmitter carried by the chamber and operatively associated with the sensor. The transmitter is arranged to send wirelessly a radiation signal indicative of the sensed condition. The system further includes a radiation signal receiver carried by the base unit and arranged to receive the radiation signal from the transmitter. The system further includes a control unit that controls the condition based at least in part on the radiation signal received by the radiation signal receiver.

These and other objects, features, and characteristics of the present invention, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention. As used in the specification and in the claims, the singular form of "a", "an", and "the" include plural referents unless the context clearly dictates otherwise.
FIG. 1 is a diagrammatic front view of a humidifier in accordance with an embodiment of the present invention;
FIG. 2 is a diagrammatic front view of a humidifier in accordance with an embodiment of the present invention;
FIG. 3 is a diagrammatic front view of a humidifier in accordance with an embodiment of the present invention;
FIG. 4 illustrates a method of operation of the present invention; and
FIG. 5 is a diagrammatic front view of a breathing system with a humidifier in accordance with an embodiment of the present invention.

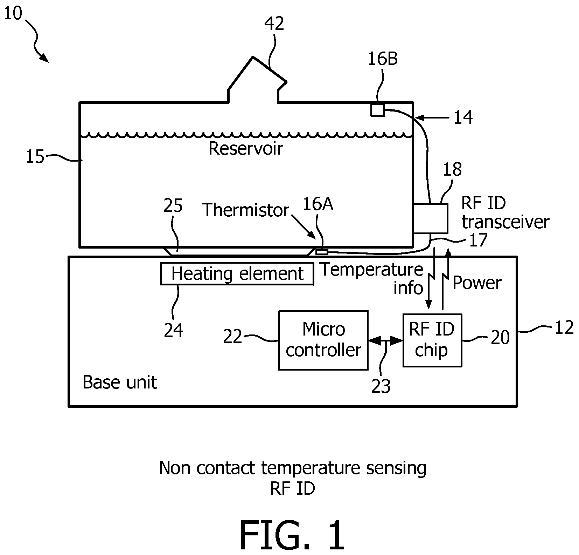

A humidifier 10 for humidifying a breathing gas for the artificial respiration of a user is described below with reference to FIG. 1. As shown in FIG. 1, humidifier 10 includes a base unit 12 and a chamber 14 constructed and arranged to hold a reservoir of liquid 15. The liquid maybe composed primarily, or entirely, of water. Sensors 16A and/or 16B are operatively connected to chamber 14 and are configured to sense a condition within the chamber. The condition may be a temperature of the liquid and/or a humidity of gas in chamber 14, or any other condition or parameter, such as, for example, a pressure of the gas within chamber 14, a flow of the gas through the chamber, an oxygen level of the gas in the chamber, and/or a carbon dioxide level of the gas in the chamber. Humidifier 10 may optionally include one or both types of sensors 16A, 16B.

In some embodiments, sensor 16B may optionally be configured to measure other conditions or parameters, such as, but not limited to, the aforementioned examples, and may be placed at various locations on or in chamber 14. In one embodiment, sensor 16B may be placed near a chamber outlet 42 of the chamber to measure the flow of gas exiting the chamber. Sensor 16B may also be a pressure sensor, a carbon dioxide sensor, or an oxygen sensor. In addition, plural sensors 16B may be provided to measure more than one of these parameters. In an optional embodiment that measures temperature, sensor 16A may be a thermistor, thermometer, thermopile, or other temperature sensing devices. Sensor 16B may be a humidity sensor in communication with the gas above the liquid in the chamber.

Chamber 14 carries a radiation signal transmitter 18 that is operatively associated with sensors 16A and/or 16B. Transmitter 18 is arranged to send a radiation signal indicative of the sensed condition. Base unit 12 carries a radiation signal receiver 20 that is arranged to receive the radiation signal from the transmitter 18. In this embodiment, transmitter 18 and/or receiver 20 comprises RFID chips. However, as disclosed below, in other embodiments, it may take the form of a secondary coil or primary coil of a transformer or an optical emitter or receiver. In other embodiments, transmitter 18 and receiver 20 may be infrared devices, Bluetooth devices, or other devices that enable contactless transmission of information between transmitter 18 and receiver 20. Humidifier 10 also includes a control unit 22, which may take the form of, for example, a microcontroller or processor, that controls the condition based at least in part on the radiation signal received by radiation signal receiver 20.

The reservoir of liquid 15 may be heated by a heating element 24 to cause the reservoir of liquid to evaporate to produce vapor within chamber 14. Increased water vapor increases the capability to provide more humidity to the air that is delivered to a user. As shown in FIG. 1, chamber 14 may include chamber outlet 42, wherein humidified gas exits from the chamber to be delivered to the user. The amount by which the humidity level of the gas is increased by humidifier 10 depends on the amount of vapor delivered to the gas as it passes through the humidifier. This is a function of, among other things, the temperature of the reservoir of liquid 15, which in turn is based at least in part on the output of heating element 24.

In some embodiments, the output of heating element 24, and thus the humidity of the gas in the chamber, may be fixed or may be set by the user by using a humidification interface (not shown) disposed on the humidifier. In some embodiments, control unit 22 may be configured to convert the humidification setting to a temperature value such that the output of heating element 24 may be controlled depending on user-selected humidity levels. The output of heating element 24 affects the temperature of the reservoir of liquid 15 and thus affects the humidity of the gas in chamber 14. In some embodiments, the user may set the temperature of the reservoir of liquid 15 using a temperature interface (not shown) disposed on humidifier 10. Control unit 22 may be configured to adjust the output of heating element 24 such that the condition within chamber 14 is substantially equal to the selected temperature of the liquid and/or the selected humidity of the gas.

In one embodiment, base unit 12 and heating element 24 may be jointly powered (e.g., via a common electrical plug or battery). Alternatively, base unit 12 and heating element 24 may be separately powered.

Heating element 24 may be an electrical heating element, microwave heater, or any other type of heat generator. In one embodiment, heating element 24 may be in contact with a thermally conductive material provided on a base plate 25 of the chamber 14 so that heat applied to base plate 25 is conducted into the liquid. In one embodiment, heating element 24 may be carried by the base unit 12. In one embodiment, heating element 24 may be removably connected to base unit 12.

As shown in FIG. 1, sensor 16A is a thermistor that is in (direct or indirect) contact with the reservoir of liquid 15 and is configured to sense the temperature of the liquid. In this embodiment, sensor 16A has an electrical resistance that varies directly or inversely with temperature. Thus, at a given temperature, sensor 16A has a certain value that can be correlated precisely to a given temperature. It is contemplated that in other embodiments a thermocouple or thermopile may be used, wherein the thermocouple or thermopile may convert a sensed temperature to an output voltage.

In this embodiment, transmitter 18 and receiver 20 may include a RFID chip. Receiver 20 may be a RFID transceiver and transmitter 18 may be a RFID transponder. Transmitter 18 may be passive and may be powered by receiver 20 when transmitter 18 is within a predetermined range of receiver 20. As such, in one embodiment, when chamber 14 is detached from base unit 12 to be cleaned and/or refilled with liquid, transmitter 18 is out of range from receiver 20 and thus is not powered by receiver 20 and does not transmit signals indicating the temperature. Transmitter 18 may optionally be an active transponder or a transceiver in some embodiments.

In the embodiment of FIG. 1, transmitter 18 is operatively connected to sensor 16A via a connector 17 such that temperature information sensed by the sensor 16A is received by the transmitter 18. In some embodiments, connector 17 may be a copper wire, a cable, or other communication links. Chamber 14 may be powered by RFID coupling.

Receiver 20 is configured to interrogate transmitter 18 for the temperature sensed by the sensor 16A, to power the transmitter, and to receive the data transmitted by the transmitter via a radiofrequency link. Transmitter 18 is configured to extract power from receiver 20 and to wirelessly transmit signals indicative of the temperature sensed by the sensor 16A via the radiofrequency link. Power may be directly or indirectly supplied from transmitter 18 to sensor 16A via aluminum or copper wires, or other links.

Receiver 20 may be operatively connected to control unit 22 via a connector 23 and may send signals to the control unit 22 indicative of the temperature of the reservoir of liquid 15 sensed by sensor 16A. In some embodiments, connector 23 may be a copper wire, a cable, or other communication links. In one embodiment, receiver 20 may be coupled to a power supply (i.e., battery, plug, A/C main, or other power sources) via wires, such as, for example, aluminum or copper wires. In one embodiment, control unit 22 may be directly connected to the power supply via a wire, and power may be provided from the control unit to receiver 20 via a wire. Control unit 22 may be a microcontroller or processor that is configured to control the temperature of the reservoir of liquid 15 based on the wireless signals indicating the temperature of reservoir 15. Control unit 22 may also be configured to process the temperature information it receives from the receiver 20 such that the temperature value and/or corresponding humidification value may be displayed to the user.

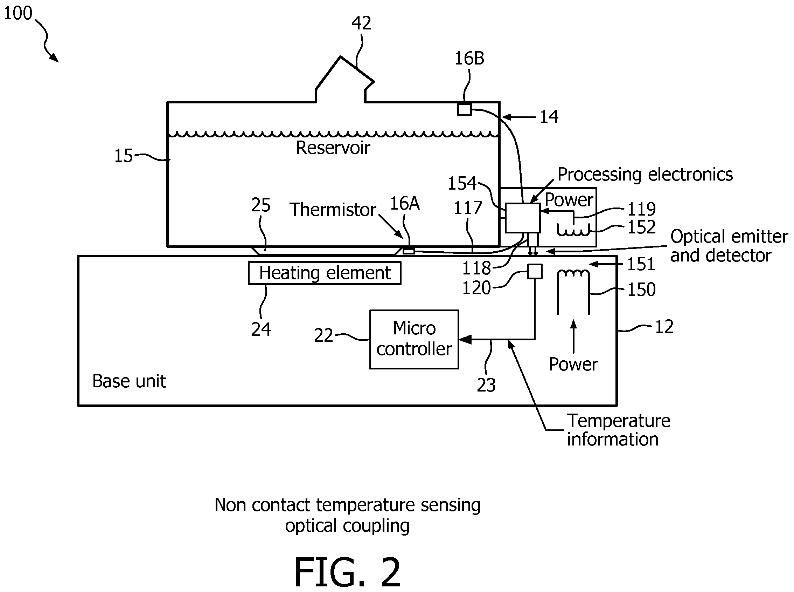

In the embodiment shown in FIG. 2, power is supplied from base unit 12 to chamber 14 using electromagnetic induction. A primary coil 150 of a transformer 151 is carried by the base unit 12 and a secondary coil 152 of transformer 151 is carried by chamber 14. Primary coil 150 may be (directly or indirectly) linked to a power supply (i.e., battery, plug, A/C main). Primary coil 150 may be coupled to the power supply via wires, such as, for example, aluminum or copper wires. In one embodiment, control unit 22 may be directly connected to a power supply via a wire, and power may be provided from the control unit to primary coil 150 via a wire. A varying current in the primary coil 150 creates a varying magnetic field in the cores, thus inducing voltage in the secondary coil 152. As such, power may be supplied to the chamber 14 contactlessly. Power may then be provided from secondary coil 152 to other components of chamber 14, such as the processing unit 154 via a wire 119, which may be, for example, an aluminum wire, a copper wire, or other wires. In one embodiment, power may be provided from secondary coil 152 to sensor 16A via aluminum or copper wires, cables, or other links. Sensor 16A may optionally be connected to an alternative power source (i.e., a battery).

Many of the components of humidifier 100 shown in the embodiment of FIG. 2 are similar to the components of the embodiment shown in FIG. 1, and thus will be labeled the same. As shown in FIG. 2, sensor 16A may be carried by chamber 14 and may be configured to detect the temperature of the reservoir of liquid 15. Similar to the embodiment of FIG. 1, humidifier 100 includes base unit 12 and chamber 14 constructed and arranged to hold the reservoir of liquid 15. Chamber 14 carries a radiation signal transmitter 118 that is operatively associated with sensor 16A. Transmitter 118 is arranged to send a radiation signal indicative of the sensed temperature. Base unit 12 carries a radiation signal receiver 120 that is arranged to receive the radiation signal from the transmitter 118. Humidifier 100 also includes a control unit 22 that controls the condition within the chamber 14, the condition being the temperature of the liquid and/or the humidity of the gas in the chamber 14, based at least in part on the radiation signal received by the radiation signal receiver 120.

In this embodiment, the sensor 16A is a thermistor. The resistance of sensor 16A varies directly or inversely with the temperature sensed by the sensor. In this embodiment, sensor 16A may be operatively connected to a processing unit 154 via a connector 117, which may take the form of a copper wire, cable, or other communication links. Processing unit 154 may include software, logic, and/or circuitry to process the signals received from sensor 16A. The processing unit may be configured to convert the resistance value of the sensor into a signal that is frequency or period dependent based on the temperature of the reservoir of liquid 15. In one embodiment, processing unit 154 may include an oscillator circuit with a variable oscillation frequency which changes in response to a change in the resistance of the sensor 16A. Processing unit 154 may be powered by secondary coil 152 of transformer 151. It is contemplated that other temperature sensors may be used instead of the thermistor, such as a thermocouple, thermopile, thermometer, and other temperature sensors known in the art.

Processing unit 154 may be operatively connected by a connector, which may take the form of a copper wire, a cable, or other communication links, to a radiation signal transmitter 118 configured to transmit signals indicative of the temperature of the reservoir of liquid 15 to a radiation signal receiver 120 disposed on base unit 12. Power may be supplied from secondary coil 152 to transmitter 118 via aluminum or copper wires, or other links. In this embodiment, radiation signal transmitter 118 is an optical emitter and radiation signal receiver 120 is an optical receiver. In one embodiment, optical emitter 118 is configured to transmit light (radiation) pulses signaling the frequency received from processing unit 154 indicative of the temperature sensed by sensor 16A. After receiving the signals from optical emitter 118, optical receiver 120 may send signals to control unit 22, which may take the form of, for example, a microcontroller or processor, wherein the signals are processed, via a connector 123. In some embodiments, connector 123 may be a copper wire, a cable, or other communication links. Power may be supplied to receiver 120 from control unit 22 or directly from a power supply via aluminum or copper wires, or other links. Control unit 22 may include logic, software, and/or circuitry to convert the frequency or period value into a temperature and/or humidity value, and the temperature value and/or corresponding humidification value may be displayed to the user.

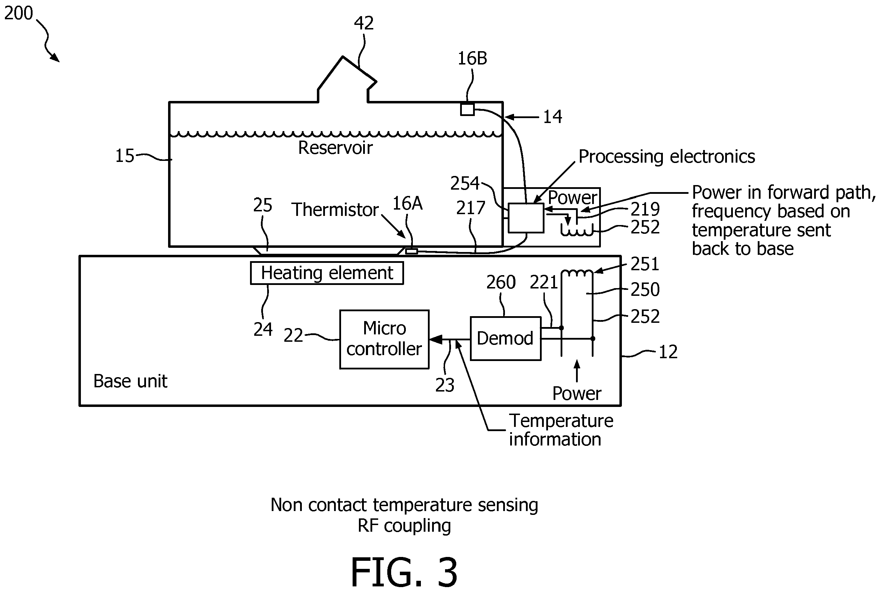

In the embodiment shown in FIG. 3, a transformer 251 is used to supply power to chamber 14 and to transmit signals indicative of the temperature of the reservoir of liquid 15. Many of the components of humidifier 200 shown in the embodiment of FIG. 3 are similar to the components of the embodiment shown in FIG. 1, and thus will be labeled the same. Similar to the embodiment of FIG. 1, humidifier 200 includes base unit 12 and chamber 14 that is constructed and arranged to hold a reservoir of liquid 15. Sensor 16A is operatively connected to reservoir 14. Reservoir 14 carries a radiation signal transmitter 252 that is operatively associated with sensor 16A. Transmitter 252 is arranged to send a radiation signal indicative of the sensed temperature. Base unit 12 carries a radiation signal receiver 250 that is arranged to receive the radiation signal from transmitter 252. Humidifier 200 also includes control unit 22 that includes software, logic, or circuitry for controlling the condition within chamber 14, the condition being the temperature of the liquid and/or the humidity of the gas in the chamber, based at least in part on the radiation signal received by radiation signal receiver 250.

In this embodiment, radiation signal transmitter 252 is a secondary coil of the transformer 251 and radiation signal receiver 250 is a primary coil of transformer 251. Primary coil 250 of transformer 251 is carried by base unit 12 and secondary coil 252 of transformer 251 is carried by chamber 14. In one embodiment, primary coil 250 may be (directly or indirectly) linked to a power supply (i.e., battery, plug, A/C main, or other power sources). Primary coil 250 may be linked to a power supply via a wire 225, which may take the form of an aluminum wire, a copper wire, or other wires. In one embodiment, control unit 22, which may take the form of, for example a microcontroller or processor, may be coupled to a power supply via a wire, and power may be provided to primary coil 250 from control unit 22 via a wire. As previously described, a varying current in primary coil 250 creates a varying magnetic field in the cores, thus inducing voltage in secondary coil 252. As such, power may be supplied to the chamber 14 contactlessly. Power may then be provided from secondary coil 252 to other components of chamber 14, such as processing unit 254 via a wire, which may be, for example, an aluminum wire, a copper wire, or other wires. In one embodiment, power may be provided from secondary coil 252 to sensor 16A via wires or other links.

In the embodiment shown in FIG. 3, sensor 16A is a thermistor that is disposed on chamber 14 and is configured to sense the temperature of the reservoir of liquid 15. As described previously, the resistance of sensor 16A varies directly or inversely with the temperature sensed by the sensor. Sensor 16A may be operatively connected to processing unit 254 configured to convert the resistance value of sensor 16A into a signal that is frequency or period dependent based on the temperature of the reservoir of liquid 15. In one embodiment, sensor 16A may be operatively connected to the processing unit 254 via a connector 217, which may take the form of a copper wire, cable, or other communication links. It is contemplated that other temperature sensors may be used instead of the thermistor, such as a thermocouple, thermopile, thermometer, and other temperature sensors known in the art.

Sensor 16A may send signals indicative of the temperature of the reservoir of liquid 15 to processing unit 254, which may include software, logic, and/or circuitry to process the signals received from the sensor 16A. In one embodiment, processing unit 254 may send signals to secondary coil 252 via a connector 219, which may be a copper wire, a cable, or other communication links. In one embodiment, processing unit 254 may be configured to convert the signals to RF signals, which may then be sent to secondary coil 252. Secondary coil 252 may then couple the RF signal indicative of the temperature of the reservoir of liquid 15 to primary coil 250. The frequency of the signals being sent to primary coil 250 may depend on the temperature sensed by sensor 16A. Primary coil 250 may be operatively connected to a demodulator 260 configured to recover the temperature information from the modulated signals via a connector 221.

In some embodiments, connector 221 may be a copper wire, a cable, or other communication links. Demodulator 260 may include software, logic, and/or circuitry to process the modulated signals. Demodulator 260 may then send the processed information via a connector 223 to control unit 22, wherein the information is further processed and the output of the heating element 24 is controlled based on the temperature of the reservoir of liquid 15 sensed by the sensor 16A. In some embodiments, connector 223 may be a copper wire, a cable, or other communication links. Control unit 22 may include software, logic, and/or circuitry to process the temperature information it receives such that the temperature value and/or corresponding humidification value may be displayed to the user.

It is contemplated that the embodiments described above and shown in FIGS. 1, 2, and 3 may include sensor 16B that may be a humidity sensor configured to sense the humidity of the gas in chamber 14. As noted above, sensor 16B may be other types of sensors configured to sense other parameters or conditions, such as, for example, the pressure of the gas within chamber 14, the flow of the gas through the chamber, the oxygen level of the gas in the chamber, and/or the carbon dioxide level of the gas in the chamber. Sensor 16B may be operatively connected to the other components of the humidifier 10, 100, or 200 in a similar manner as sensor 16A. Humidifier 10, 100, or 200 may include one or both sensors 16A and/or 16B. In embodiments that include sensor 16B, transmitter 18, 118, or 252 may be arranged to send a radiation signal indicative of the sensed condition. Receiver 20, 220, or 250 may be arranged to receive the radiation signal from transmitter 18, 118, or 252, and the control unit 22 may control the condition within chamber 14 based at least in part on the radiation signal received by receiver 20, 220, or 250.

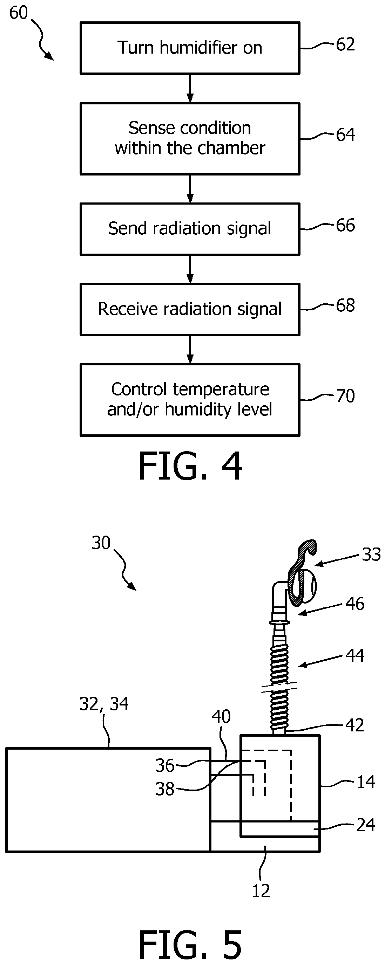

FIG. 4 is illustrates a method 60 of operation of the humidifier. In procedure 62, humidifier 10 is turned on. The method proceeds to procedure 64, wherein sensor 16A, 16B senses the condition within chamber 14, such as the temperature of the liquid, the humidity of the gas in the chamber, and/or other conditions or parameters. In embodiments that include sensor 16A and wherein sensor 16A is a thermistor, the resistance of the sensor varies according to the temperature. As such, the resistance value of the sensor is indicative of the temperature of the reservoir of liquid 15. In embodiments that include sensor 16B and wherein sensor 16B is a humidity sensor, the resistance of sensor 16B varies according to the humidity. As such, the resistance value of the sensor is indicative of the humidity of the gas in chamber 14. This information is sent to the radiation signal transmitter 18, 118, or 252. In embodiments wherein a processing unit 154, 254 is included, signals are sent to the processing unit for processing before being sent to radiation signal transmitter 18, 118, or 252. In some embodiments, the signals may be amplified or converted to RF frequency before they are sent to radiation transmitter 18, 118, or 252.

Method 60 proceeds to procedure 66, wherein radiation signal transmitter 18, 118, or 252 transmits the signals indicative of the sensed condition within chamber 14 to radiation signal receiver 20, 120, or 250. Method 60 then proceeds to procedure 68, wherein the signals are received by radiation signal receiver 20, 120, or 250. During this process, radiation signal receiver 20, 120, or 250 may be used to provide power to radiation signal transmitter 18, 118, or 252. In the embodiments shown in FIG. 2 and 3, radiation signal receiver 20, 120, 250 provides power to radiation signal transmitter 18, 118, or 252 using electromagnetic induction.

The method then proceeds to procedure 70, wherein the signals are sent to the control unit 22 to be processed. In the embodiment of FIG. 3, the signals are first sent to the demodulator 260 wherein information is extracted from the modulated signals to be sent to the control unit 22. After processing the signals or information into a temperature value or humidity value, control unit 22 then controls the temperature of the liquid and/or the humidity of the gas in chamber 14 based at least in part on the wireless radiation signal received by radiation signal receiver 20, 120, or 250. The temperature and/or humidity may be controlled by controlling the output of heating element 24. In some embodiments, the temperature value and/or corresponding humidification value may be displayed to the user.

FIG. 5 illustrates a breathing system 30 using humidifier 10, 100, or 200. System 30 includes a source of compressed gas 32, humidifier 10, 100, or 200, and a user interface 33. As shown in FIG. 5, the source of compressed gas 32 comprises a PAP machine 34, as is well known in the art (e.g., Respironics' REMstar® and SleepEasy® lines of PAP machines). However, according to alternative embodiments of the present invention, a variety of alternative sources of compressed gas 32 may be used without deviating from the scope of the present invention (e.g., ventilator, oxygen concentrator, compressed gas tank, etc.) to provide a variety of compressed gases (e.g., compressed air from the ambient environment or a storage container, oxygen-enriched air, etc.). A compressed gas outlet 36 of PAP machine 34 fluidly connects to a chamber inlet 38 of the humidifier 14 via a suitable passageway (e.g., tubing 40).

As shown in FIG. 5, chamber outlet 42 of the humidifier 10, 100, or 200 fluidly connects to the user interface 33 via a suitable fluid passageway (e.g., flexible tubing 44). User interface 33 may comprise any type of suitable user interface for directing compressed gas toward and/or into a user's airway (e.g., full face mask; nasal mask; nasal pillows mask; intubation tube). Depending on the type of breathing system used, user interface 33 may include an exhalation port 46 (e.g., a check valve or relief valve that opens to the ambient environment when a pressure within the user interface exceeds a predetermined value).

U.S. Patent Nos. 5,655,522, 6,360,741, 6,401,713, 6,467,477, 6,644,311, and 6,959,710 disclose a variety of sources of compressed gas, user interfaces, and/or other parts of breathing systems that maybe used as part of one or more embodiments of the present invention.

Base unit 12 releasably attaches to the PAP machine 34. However, base unit 12 may alternatively be permanently attached to PAP machine 34 (e.g., via integral formation) without deviating from the scope of the present invention. The attachment (releasable or not) between base unit 12 and PAP machine 34 facilitates secure connection between the PAP machine's compressed gas outlet 36 and inlet 38 of humidifier 10 (e.g., via suitable tubing 40). Alternatively, humidifier 10 may comprise a stand-alone unit that connects to the PAP machine 34 via a suitable passageway (e.g., flexible tube).

In some embodiments, PAP machine 34 and base unit 12 may be jointly powered (e.g., via a common electrical plug or battery). Alternatively, in some embodiments, PAP machine 34 and base unit 12 may be separately powered.

As shown in FIG. 5, heating element 24 mounts to base unit 12 and is positioned relative to the reservoir of liquid 15 of chamber 14 so as to heat liquid therein to facilitate evaporation of the heated liquid and humidification of the gas passing through chamber 14. Alternatively, in some embodiments, the heating element 24 may be incorporated into chamber 14, itself.

As the gas in system 30 passes through chamber 14 from inlet 38 to outlet 42, the vapor in chamber 14 is delivered to the gas, thereby elevating the humidity of the gas in the system downstream from humidifier 10, 100, or 200.

Hereinafter, operation of system 30 is described. Prior to use, a user detaches humidification chamber 14 from the system and fills it to a desired level with a suitable humidification liquid (e.g., distilled water). The user then turns system 30 on. The user may set a desired humidification level of the system using the humidification interface. In one embodiment, the user may set a desired temperature setting using a temperature interface.

As shown in FIG. 5, PAP machine 34 directs compressed gas into the chamber 14 via the PAP machine's chamber outlet 36, tubing 40, and chamber's chamber inlet 38. Heating element 24 heats the liquid, causing the liquid to evaporate into the compressed gas stream within chamber 14. The gas pressure directs the humidified gas to user interface 33 via the humidification chamber's outlet 42 and tubing 44, thereby providing compressed, humidified gas to the user. During this process, sensors 16A and/or 16B senses the condition within chamber 14, such as the temperature of the liquid and/or the humidity of the gas in the chamber. In embodiments wherein sensor 16A is a thermistor, the resistance of sensor 16A may vary according to the varying temperature of the reservoir of liquid 15. The resistance value may be transmitted to transmitter 18, 118, or 252. Receiver 20, 120, or 250 provides power to transmitter 18, 118, or 252 and the transmitter 18, 118, or 252 transmits signals indicative of the condition within chamber 14 to receiver 20, 120, or 250 via the "airspace" between transmitter 18, 118, or 252 and receiver 20, 120, or 250.

After receiving the temperature or humidity information from transmitter 18, 118, or 252, receiver 20, 120, or 250 sends the information to the control unit 22, which processes the signals indicative of the temperature or humidity and controls the condition within chamber 14 based on a comparison of the predetermined user-desired temperature or humidification value and the current condition within the chamber 14.

The current temperature value and/or corresponding humidification value may also be displayed by humidifier 10, 100, or 200.

Although the invention has been described in detail for the purpose of illustration based on what is currently considered to be the most practical and preferred embodiments, it is to be understood that such detail is solely for that purpose and that the invention is not limited to the disclosed embodiments, but, on the contrary, is intended to cover modifications and equivalent arrangements that are within the scope of the appended claims. For example, it is to be understood that the present invention contemplates that, to the extent possible, one or more features of any embodiment can be combined with one or more features of any other embodiment.

## Claims

1. A respiration humidifier (10) comprising:
a base unit (12);
a chamber (14) constructed and arranged to hold a reservoir of liquid;
a radiation signal transmitter (18) carried by the chamber (14) and operatively associated with a sensor (16A, 16B) carried by the chamber (14), the transmitter arranged to wirelessly send a radiation signal indicative of a condition sensed by the sensor;
a radiation signal receiver (20) carried by the base unit and arranged to receive the radiation signal from the transmitter; and
a control unit (22) that controls the condition based at least in part on the radiation signal received by the radiation signal receiver,
**characterized in that**
- the chamber (14) is a detachable chamber;
- the sensor (16A,16B) is operatively configured to sense a condition within the chamber (14), wherein the sensor (16A,16B) is a temperature sensor separate from the transmitter (18) and operatively connected at a distance to the radiation signal transmitter (18) via a connector (17, 217);
- the condition is a temperature of the liquid or a humidity of gas in the chamber (14); and
- the transmitter (18) is arranged to be contactlessly powered by the receiver when the chamber (14) is attached to the base unit (12) and the transmitter (18) is in a predetermined range from the receiver (20) and to be powered off when the chamber (14) is detached from the base unit (12) and the transmitter is out of the range.

2. A respiration humidifier (10) according to claim 1, wherein power is provided from the radiation signal transmitter (18; 252) to the sensor (16A) via the connector (17; 217).

3. The humidifier (10) of claim 1, wherein the radiation signal transmitter (18) is a RFID chip, an optical receiver, or a primary coil of a transformer.

4. The humidifier (10) of claim 1, wherein the radiation signal receiver is a RFID chip, an optical receiver, or a secondary coil of a transformer

5. The humidifier (10) of claim 1, wherein power is provided to the chamber by inductive coupling or by RFID coupling.

6. The humidifier (10) of claim 1, further comprising an additional sensor that senses an additional condition within the chamber.

7. A method for sensing a condition of a respiration humidifier (10) comprising a base unit (12) and a chamber (14) constructed and arranged to hold a reservoir of liquid, the method comprising:
sensing (64) the condition within the chamber via a sensor (16A, 16B) carried by the chamber (14); sending (66) wirelessly a radiation signal indicative of the sensed condition with a radiation signal transmitter (18) carried by the chamber;
receiving (68) the radiation signal indicative of the sensed condition by a radiation signal receiver (20); and
controlling (70) the condition based at least in part on the radiation signal received by the radiation signal receiver,
**characterized in that**
- the chamber (14) is a detachable chamber;
- the sensor (16A,16B) is operatively configured to sense a condition within the chamber, wherein the sensor (16A,16B) is a temperature sensor separate from the transmitter (18) and operatively connected at a distance to the radiation signal transmitter (18) via a connector (17, 217);
- the condition is a temperature of the liquid or a humidity of gas in the chamber; and
- the transmitter (18) is arranged to be contactlessly powered by the receiver when the chamber (14) is attached to the base unit (12) and the transmitter (18) is in a predetermined range from the receiver (20) and to be powered off when the chamber (14) is detached from the base unit (12) and the transmitter is out of the range.

8. The method of claim 7, wherein the radiation signal transmitter is a RFID chip, an optical receiver, or a primary coil of a transformer.

9. The method of claim 7, wherein the radiation signal receiver is a RFID chip, an optical receiver, or a secondary coil of a transformer.

10. The method of claim 7, wherein power is provided to the chamber by inductive coupling or RFID coupling.

11. A system (30) for delivering a flow of gas to an airway of a user, the system comprising:
a gas flow generator (32, 34) that generates a flow of gas;
a user circuit (44, 46) coupled to the gas flow generator and adapted to communicate the flow of gas to an airway of a user; and
a humidifier (14) according to claim 1 operatively connected to the user circuit to elevate the humidity of the gas in the user circuit to a circuit humidity level.

12. The system (30) of claim 11, further comprising an additional sensor that senses an additional condition within the chamber.

## Patentansprüche

1. Atmungsbefeuchter (10), umfassend:
eine Basiseinheit (12);
eine Kammer (14), die derart ausgelegt und angeordnet ist, um einen Flüssigkeitstank zu halten;
einen Strahlungssignalgeber (18), der von der Kammer (14) getragen ist und operativ an einen Sensor (16A, 16B), der von der Kammer (14) getragen ist, angegliedert ist, wobei der Signalgeber derart angeordnet ist, um ein Strahlungssignal drahtlos zu senden, was ein Hinweis auf einen von dem Sensor erfassten Zustand ist;
einen Strahlungssignalempfänger (20), der von der Basiseinheit getragen und derart angeordnet ist, um das Strahlungssignal von dem Signalgeber zu empfangen; und
eine Steuereinheit (22), die den Zustand wenigstens teilweise basierend auf dem von dem Strahlungssignalempfänger empfangenen Strahlungssignal zu steuern,
**dadurch gekennzeichnet, dass**
- die Kammer (14) eine abnehmbare Kammer ist
- der Sensor (16A, 16B) operativ derart konfiguriert ist, um einen Zustand innerhalb der Kammer (14) zu erfassen, wobei der Sensor (16A, 16B) ein Temperatursensor separat von dem Signalgeber (18) ist und über einen Anschluss (17, 217) in einem Abstand zu dem Strahlungssignalgeber (18) operativ verbunden ist;
- der Zustand eine Temperatur der Flüssigkeit oder eine Feuchtigkeit von Gas in der Kammer (14) ist; und
- der Signalgeber (18) derart angeordnet ist, um drahtlos von dem Empfänger eingeschaltet zu werden, wenn die Kammer (14) an der Basiseinheit (12) befestigt ist und sich der Signalgeber (18) in einer vorbestimmten Reichweite des Empfängers (20) befindet, und ausgeschaltet zu werden, wenn die Kammer (14) von der Basiseinheit (12) abgenommen ist, und sich der Signalgeber außerhalb der Reichweite befindet.

2. Atmungsbefeuchter (10) nach Anspruch 1, wobei Strom von dem Strahlungssignalgeber (18; 252) über den Anschluss (17; 217) an den Sensor (16A) bereitgestellt wird.

3. Befeuchter (10) nach Anspruch 1, wobei der Strahlungssignalgeber (18) ein RFID-Chip, ein optischer Empfänger, oder eine Primärspule eines Stromwandlers ist.

4. Befeuchter (10) nach Anspruch 1, wobei der Strahlungssignalempfänger ein RIFD-Chip, ein optischer Empfänger oder eine Primärspule eines Stromwandlers ist.

5. Befeuchter (10) nach Anspruch 1, wobei Strom durch induktive Kopplung oder durch RFID-Kopplung an die Kammer bereitgestellt wird.

6. Befeuchter (10) nach Anspruch 1, ferner umfassend einen zusätzlichen Sensor, der einen zusätzlichen Zustand innerhalb der Kammer erfasst.

7. Verfahren zum Erfassen eines Zustands eines Atmungsbefeuchters (10), umfassend eine Basiseinheit (12) und eine Kammer (14), die derart ausgelegt und angeordnet ist, um einen Flüssigkeitstank zu halten, wobei das Verfahren Folgendes umfasst:
das Erfassen (64) des Zustands innerhalb der Kamera über einen Sensor (16A, 16B), der von der Kammer (14) getragen ist;
das drahtlose Senden (66) eines Strahlungssignals, was ein Hinweis auf den erfassten Zustand ist, mit einem Strahlungssignalgeber (18), der von der Kammer getragen ist;
das Empfangen (68) des Strahlungssignals, was ein Hinweis auf den von einem Strahlungssignalempfänger (20) erfassten Zustand ist; und
das Steuern (70) des Zustands wenigstens teilweise basierend auf dem von dem Strahlungssignalempfänger empfangenen Strahlungssignal,
**dadurch gekennzeichnet, dass**
- die Kammer (14) eine abnehmbare Kammer ist;
- der Sensor (16A, 16B) derart operativ konfiguriert ist, um einen Zustand innerhalb der Kammer zu erfassen, wobei der Sensor (16A, 16B) ein Temperatursensor separat von dem Empfänger (18) ist und über einen Anschluss (17, 217) in einem Abstand zu dem Strahlungssignalgeber (18) operativ verbunden ist;
- der Zustand eine Temperatur der Flüssigkeit oder eine Feuchtigkeit von Gas in der Kammer ist; und
- der Signalgeber (18) derart angeordnet ist, um drahtlos von dem Empfänger eingeschaltet zu werden, wenn die Kammer (14) an der Basiseinheit (12) befestigt ist und sich der Signalgeber (18) in einer vorbestimmten Reichweite des Empfängers (20) befindet, und ausgeschaltet zu werden, wenn die Kammer (14) von der Basiseinheit (12) abgenommen ist, und sich der Signalgeber außerhalb der Reichweite befindet.

8. Verfahren nach Anspruch 7, wobei der Strahlungssignalgeber ein RIFD-Chip, ein optischer Empfänger oder eine Primärspule eines Stromwandlers ist.

9. Verfahren nach Anspruch 7, wobei der Strahlungssignalempfänger ein RIFD-Chip, ein optischer Empfänger oder eine Primärspule eines Stromwandlers ist.

10. Verfahren nach Anspruch 7, wobei Strom durch induktive Kopplung oder durch RFID-Kopplung an die Kammer bereitgestellt wird.

11. System (30) zur Abgabe eines Gasstroms an eine Luftröhre eines Benutzers, wobei das System Folgendes umfasst:
einen Gasstromgenerator (32, 34), der einen Gasstrom erzeugt;
einen Benutzerschaltkreis (44, 46), der an den Gasstromgenerator gekoppelt und derart angepasst ist, um den Gasstrom an eine Luftröhre eines Benutzers zu kommunizieren; und
einen Befeuchter (14) nach Anspruch 1, der mit dem Benutzerschaltkreis operativ verbunden ist, um die Feuchtigkeit des Gases in dem Benutzerschaltkreis auf einen Feuchtigkeitswert des Schaltkreises zu erhöhen.

12. System (30) nach Anspruch 11, ferner umfassend einen zusätzlichen Sensor, der einen zusätzlichen Zustand innerhalb der Kammer erfasst.

## Revendications

1. Humidificateur à respiration (10) comprenant :
une unité de base (12) ;
une chambre (14) construite et agencée pour contenir un réservoir de liquide ;
un émetteur de signal de rayonnement (18) porté par la chambre (14) et fonctionnellement associé à un capteur (16A, 16B) porté par la chambre (14), l'émetteur étant agencé pour envoyer sans fil un signal de rayonnement indicatif d'un état détecté par le capteur ;
un récepteur de signal de rayonnement (20) porté par l'unité de base et agencé pour recevoir le signal de rayonnement provenant de l'émetteur ; et
une unité de commande (22) qui commande l'état sur la base au moins en partie du signal de rayonnement reçu par le récepteur de signal de rayonnement,
**caractérisé en ce que**
- la chambre (14) est une chambre détachable ;
- le capteur (16A, 16B) est fonctionnellement configuré pour détecter un état dans la chambre (14), dans lequel le capteur (16A, 16B) est un capteur de température séparé de l'émetteur (18) et fonctionnellement connecté à une distance à l'émetteur de signal de rayonnement (18) par le biais d'un connecteur (17, 217) ;
- l'état est une température du liquide ou une humidité de gaz dans la chambre (14) ; et
- l'émetteur (18) est agencé pour être alimenté sans contact par le récepteur quand la chambre (14) est attachée à l'unité de base (12) et l'émetteur (18) est dans une plage prédéterminée par rapport au récepteur (20) et pour être éteint quand la chambre (14) est détachée de l'unité de base (12) et l'émetteur est en dehors de la plage.

2. Humidificateur à respiration (10) selon la revendication 1, dans lequel la puissance est fournie depuis l'émetteur de signal de rayonnement (18 ; 252) au capteur (16A) par le biais du connecteur (17 ; 217).

3. Humidificateur (10) selon la revendication 1, dans lequel l'émetteur de signal de rayonnement (18) est une puce RFID, un récepteur optique ou une bobine primaire d'un transformateur.

4. Humidificateur (10) selon la revendication 1, dans lequel le récepteur de signal de rayonnement est une puce RFID, un récepteur optique ou une bobine secondaire d'un transformateur.

5. Humidificateur (10) selon la revendication 1, dans lequel la puissance est fournie à la chambre par couplage inductif ou par couplage RFID.

6. Humidificateur (10) selon la revendication 1, comprenant en outre un capteur supplémentaire qui détecte un état supplémentaire dans la chambre.

7. Procédé pour détecter un état d'un humidificateur à respiration (10) comprenant une unité de base (12) et une chambre (14) construite et agencée pour contenir un réservoir de liquide, le procédé comprenant :
la détection (64) de l'état dans la chambre par le biais d'un capteur (16A, 16B) porté par la chambre (14) ;
l'envoi (66) sans fil d'un signal de rayonnement indicatif de l'état détecté avec un émetteur de signal de rayonnement (18) porté par la chambre,
la réception (68) du signal de rayonnement indicatif de l'état détecté par un récepteur de signal de rayonnement (20) ; et
la commande (70) de l'état sur la base au moins en partie du signal de rayonnement reçu par le récepteur de signal de rayonnement,
**caractérisé en ce que**
- la chambre (14) est une chambre détachable ;
- le capteur (16A, 16B) est fonctionnellement configuré pour détecter un état dans la chambre, dans lequel le capteur (16A, 16B) est un capteur de température séparé de l'émetteur (18) et fonctionnellement connecté à une distance à l'émetteur de signal de rayonnement (18) par le biais d'un connecteur (17, 217) ;
- l'état est une température du liquide ou une humidité de gaz dans la chambre ; et
- l'émetteur (18) est agencé pour être alimenté sans contact par le récepteur quand la chambre (14) est attachée à l'unité de base (12) et l'émetteur (18) est dans une plage prédéterminée par rapport au récepteur (20) et pour être éteint quand la chambre (14) est détachée de l'unité de base (12) et l'émetteur est en dehors de la plage.

8. Procédé selon la revendication 7, dans lequel l'émetteur de signal de rayonnement est une puce RFID, un récepteur optique ou une bobine primaire d'un transformateur.

9. Procédé selon la revendication 7, dans lequel le récepteur de signal de rayonnement est une puce RFID, un récepteur optique ou une bobine secondaire d'un transformateur.

10. Procédé selon la revendication 7, dans lequel la puissance est fournie à la chambre par couplage inductif ou par couplage RFID.

11. Système (30) pour distribuer un écoulement de gaz aux voies respiratoires d'un utilisateur, le système comprenant :
un générateur d'écoulement de gaz (32, 34) qui génère un écoulement de gaz ;
un circuit utilisateur (44, 46) couplé au générateur d'écoulement de gaz et adapté pour communiquer l'écoulement de gaz aux voies respiratoires d'un utilisateur ; et
un humidificateur (14) selon la revendication 1 fonctionnellement connecté au circuit utilisateur afin d'élever l'humidité du gaz dans le circuit utilisateur à un niveau d'humidité de circuit.

12. Système (30) selon la revendication 11, comprenant en outre un capteur supplémentaire qui détecte un état supplémentaire dans la chambre.
